(19) Europäisches Patentamt
European Patent Office
Office européen des brevets

(11) Veröffentlichungsnummer: **0 012 722**
**B1**

# (12) EUROPÄISCHE PATENTSCHRIFT

(45) Veröffentlichungstag der Patentschrift: **09.12.81**

(51) Int. Cl.³: **C 07 C 61/40,** C 07 C 69/743, C 07 C 143/20, C 07 B 19/00 // C07C49/457, C07B20/00

(21) Anmeldenummer: **79810176.2**

(22) Anmeldetag: **10.12.79**

(54) **Verfahren zur Herstellung von optisch aktiven, in 3-Stellung substituierten 2-(2',2'-Dihalogenvinyl)-cyclopropan-1-carbonsäuren und deren Derivaten; 4-(2',2',2'-Trihalogenäthyl)-cyclobutan-1-sulfonsäuresalze und deren Herstellung.**

(30) Priorität: **15.12.78 CH 12784/78**

(43) Veröffentlichungstag der Anmeldung: **25.06.80 Patentblatt 80/13**

(45) Bekanntmachung des Hinweises auf die Patenterteilung: **09.12.81 Patentblatt 81/49**

(84) Benannte Vertragsstaaten: **AT BE CH DE FR GB IT NL SE**

(56) Entgegenhaltungen:
DE-A-2813337
DE-A-2654062

HOUBEN WEYL: «Methoden der organischen Chemie», Band IV/2, IV, Auflage 1955, G. Thieme Verlag, Stuttgart, DE, «Allgemeine chemische Methoden» Seiten 519 bis 523

HOUBEN WEYL: «Methoden der organischen Chemie», Band IV/2, IV, Auflage 1955, G. Thieme Verlag, Stuttgart, DE, «Allgemeine chemische Methoden» Seiten 528 bis 529

B.L. ELIEL: «Stereochemistry of Cabon Compounds», 1962, Mc. GRAW-HILL, BOOK COMPANY INC., NEW YORK, US, Pages 56, 57

(73) Patentinhaber: **CIBA-GEIGY AG, Patentabteilung Postfach, CH-4002 Basel (CH)**

(72) Erfinder: **Dingwall, John Grey, Dr., 23 Wood Road, Brooklands Sale, Cheshire (GB)**
Erfinder: **Greuter, Hans, Dr., 75, Cos Cob Avenue, Cos Cob, Connecticut 06807 (US)**
Erfinder: **Martin, Pierre, Dr., Meisenweg 38, CH-4310 Rheinfelden (CH)**
Erfinder: **Ackermann, Peter, Dr., Reichensteinerstrasse 12, CH-4153 Reinach (CH)**
Erfinder: **Gsell, Laurenz, Dr., Maiengasse 56, CH-4056 Basel (CH)**

EP 0 012 722 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Verfahren zur Herstellung von optisch aktiven, in 3-Stellung substituierten 2-(2',2'-Dihalogenvinyl)-cyclopropan-1-carbonsäuren und deren Derivaten; 4-(2',2',2'-Trihalogenäthyl)-cyclobutan-1-sulfonsäuresalze und deren Herstellung.

Die vorliegende Erfindung betrifft ein neues Verfahren zur Herstellung von optisch aktiven, in 3-Stellung substituierten 2-(2',2'-Dihalogenvinyl)-cyclopropan-1-carbonsäuren und Derivaten davon sowie neue 4-(2',2',2'-Trihalogenäthyl)-cyclobutan-1-sulfonsäuresalze. Die genannten Cyclopropancarbonsäuren bzw. deren Derivate stellen wertvolle Ausgangsprodukte zur Herstellung von Schädlingsbekämpfungsmitteln, besonders Insektiziden, dar oder können direkt als Schädlingsbekämpfungsmittel eingesetzt werden.

Es ist allgemein bekannt, dass bei biologisch aktiven chemischen Substanzen mit einem optisch aktiven Zentrum in der Regel nur eines der beiden möglichen Spiegelbilder biologisch aktiv, das andere aber inaktiv ist.

Die bekannten insektiziden Pyrethroide sind chemische Substanzen, die mehrere solche optisch aktive Zentren besitzen. Die Literatur zeigt [vgl. M. Elliott: „Synthetic Pyrethroids", ACS Symposium Series *42*, Am. Chem. Soc. 2-9 (1971)], dass auch hier die Konfiguration an diesen Zentren massgebend die insektizide Aktivität bestimmt. Es besteht daher ein dringendes Bedürfnis für einfache wirtschaftliche Verfahren, die es ermöglichen, Pyrethroide mit der optimal wirksamen (insektiziden) Konfiguration zu synthetisieren. Damit wird erreicht, dass nur die biologisch wirksamen Komponenten eines Pyrethroidpräparates Anwendung finden. Aufwandmengen und Belastung der Umwelt werden hierdurch weit geringer als bei der Anwendung von Pyrethroidpräparaten, die auch weniger aktive oder gänzlich unwirksame Isomere erhalten.

Aus der Literatur sind verschiedene Verfahren zur Herstellung von optisch aktiven 2-(2',2'-Dihalogenvinyl)-3,3-dimethylcyclopropan-1-carbonsäuren bekannt, die aber den obigen Anforderungen nicht voll zu genügen vermögen. Die japanischen Offenlegungsschriften 50-131.953, 51-36.441 und 51-143.647 offenbaren Verfahren zur Racematspaltung der trans-2-(2',2'-Dichlorvinyl)-3,3-dimethylcyclopropancarbonsäure oder Gemischen der trans- und cis- Säure durch Umsetzung mit bestimmten optisch aktiven Aminen und Zersetzung der erhaltenen reinen diastereomeren Salze. Ferner ist aus der deutschen Offenlegungsschrift 2.628.477 bekannt, dass man optisch aktive 2-(2',2'-Dichlorvinyl)-3,3-dimethylcyclopropancarbonsäuren mit unerwünschter, d.h. biologisch weniger wirksamer Konfiguration, besonders das (−)-trans-Isomere, durch UV-Belichtung in Gegenwart von Photosensibilisatoren racemisieren kann. Die Ausbeuten an dem gewünschten Racemat sind jedoch gering, und ausserdem überwiegt darin der Anteil an trans- Säure stark, so dass sich dieses Verfahren nicht zur Herstellung von cis-Säuren eignet, die bei gewissen Anwendungen grössere Wirksamkeit zeigen und daher besonders bervorzugt sind.

Gemäss der britischen Patentschrift 1.446.304 können optisch aktive 2-(2',2'-Dihalogenvinyl)-3,3-dimethylcyclopropancarbonsäuren und deren Alkylester auch nach der Wittig-Reaktion ausgehend von optisch aktiven Carbonaldehydderivaten hergestellt werden. Die dafür benötigten optisch aktiven Carbonaldehydderivate sind jedoch schwer zugänglich, indem sie durch Ozonolyse entweder aus der nicht in beliebiger Menge erhältlichen natürlichen Chrysanthemumsäure oder aber aus synthetischer Chrysanthemumsäure hergestellt werden müssen. Bei der Racemisierung der Säure oder von deren Derivaten mit der unerwünschten Konfiguration entstehen hohe Anteile an Produkten mit der trans-Konfiguration.

Schliesslich können optisch aktive Cyclopropancarbonsäureester gemäss der japanischen Offenlegungsschrift 50-160.241 auch durch Umsetzung geeigneter Olefine mit Diazoessigester in Gegenwart von optisch aktiven Katalysatoren (Carbon-Addition) hergestellt werden. Die Ausbeuten an optisch aktiven Cyclopropancarbonsäureestern sind jedoch unbefriedigend. Ausserdem ist die Handhabung des zur Reaktion benötigten Diazoessigesters mit Gesundheits- und Unfallrisiken verbunden.

Es wurde nun ein Verfahren gefunden, das es erlaubt, optisch aktive, in 3-Stellung substituierte 2-(2',2'-Dihalogenvinyl)-cyclopropancarbonsäuren und Derivate davon mit hohen Anteilen an cis-Isomeren auf einfachere, wirtschaftlichere Weise und unter Vermeidung der obigen Nachteile zugänglich zu machen.

Gegenstand der Erfindung ist somit ein Verfahren zur Herstellung von optisch aktiven Cyclopropancarbonsäurederivaten der Formel I oder I'

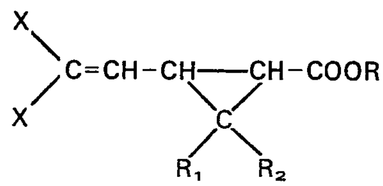
(I)

oder 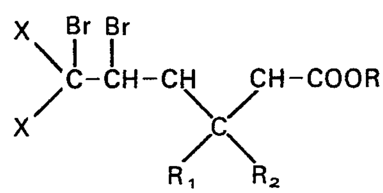
(I')

worin X Chlor oder Brom, einer der Reste $R_1$ und $R_2$ Methyl und der andere Wasserstoff oder Methyl oder $R_1$ und $R_2$ zusammen Alkylen mit 2-4 Kohlenstoffatomen und R Wasserstoff, Alkyl mit 1-4 Kohlenstoffatomen oder eine Gruppe a)

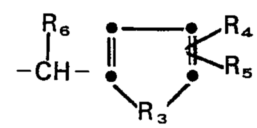
(a)

bedeutet, wobei $R_3$ Sauerstoff, Schwefel oder Vinylen, $R_4$ Wasserstoff, Methyl-, Benzylphenoxy, 4-Methylphenoxy, 4-Chlorphenoxy, 4-Fluorphenoxy oder Phenylmercapto $R_5$ Wasserstoff,

Fluor, Chlor oder Methyl und $R_6$ Wasserstoff Cyano oder Äthinyl bedeuten, indem man ein Racemat eines Cyclobutanons der Formel II

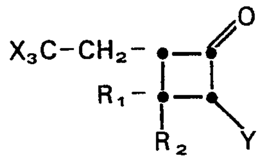

(II)

in welcher X, $R_1$ und $R_2$ die unter Formel I angegebene Bedeutung haben und Y Chlor, Brom oder eine Gruppe $-OSO_2R'$ bedeutet, wobei R' Alkyl, Halogenalkyl, Benzyl, Naphthyl oder gegebenenfalls substituiertes Phenyl darstellt, durch Umsetzung mit einem schwefeligsauren Salz einer optisch aktiven Base A in ein Gemisch von diastereomeren sulfonsauren Salzen der Formel III

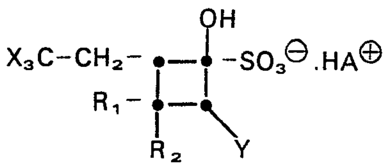

(III)

in welcher X, $R_1$, $R_2$ und Y die oben angegebene Bedeutung haben und A für eine optisch aktive Base steht, überführt, dieses Gemisch in die reinen diastereomeren sulfonsauren Salze der Formel III auftrennt und entweder direkt die reinen diastereomeren sulfonsauren Salze der Formel III oder die durch Zersetzung aus diesen erhaltenen optisch aktiven Cyclobutanone der Formel II in Gegenwart einer Base in ein optisch aktives Cyclopropancarbonsäurederivat der Formel I umwandelt und dieses gegebenenfalls durch Bromierung in ein optisch aktives Cyclopropancarbonsäurederivat der Formel I' überführt.

Die Umsetzung der Racemate von Cyclobutanonen der Formel II mit einem schwefligsaurem Salz einer optisch aktiven Base A wird zweckmässig in Gegenwart eines inerten organischen Lösungsmittels bei Temperaturen zwischen etwa 0 und 100°C und bevorzugt zwischen etwa 20 und 60°C vorgenommen.

Geeignete inerte organische Lösungsmittel sind z.B. gegebenenfalls nitrierte oder halogenierte aliphatische, cycloaliphatische oder aromatische Kohlenwasserstoffe, wie n-Hexan, n-Pentan, Cyclohexan, Benzol, Toluol, Xylole, Nitrobenzol, Chloroform, Tetrachlorkohlenstoff, Trichloräthylen, 1,1,2,2,-Tetrachloräthan, Nitromethan, Chlorbenzol, Dichlorbenzole und Trichlorbenzole;

— niedere aliphatische Alkohole, z.B. solche mit bis zu 6 C-Atomen, wie Methanol, Äthanol, Propanol, Isopropanol, Butanole und Pentanole;

— aliphatische Diole, wie Äthylenglykol und Diäthylenglykol;

— Athylenglykol- und Diäthylenglykolmono- und -dialkyläther mit je 1-4 C-Atomen in den Alkylteilen, wie Äthylenglykolmonomethyl-, -monoäthyl-, -dimethyl- und -diäthyläther, Diäthylenglykolmonomethyl- und -monoäthyl-, -dimethyl- und -diäthyläther;

— cyclische Amide, wie N-Methyl-2-pyrrolidon, N-Acetyl-2-pyrrolidon und N-Methyl-ε-caprolactam;

— Amide der Kohlensäure, wie Tetramethylharnstoff und Dimorpholinocarbonyl;

— Amide der phosphorigen Säure, der Phosphorsäure, der Phenylphosphonsäure oder von aliphatischen Phosphonsäuren mit 1-3 C-Atomen im Säureteile, wie Phosphorsäuretriamid, Phosphorsäure-tris(dimethylamid), Phosphorsäuretrimorpholid, Phosphorsäuretripyrrolinid, Phosphorsäure-bis(dimethylamid)-morpholid, Phosphorsäuredimethylamiddiäthylamidmorpholid, Phosphorigsäure-tris (dimethylamid), Tetramethyldiamid der Methanphosphonsäure;

— Amide der Schwefelsäure, von aliphatischen oder aromatischen Sulfonsäuren, wie Tetramethylsulfamid, Dimethylamid der Methansulfonsäure oder p-Toluolsulfonsäureamid;

— schwefelhaltige Lösungsmittel, wie organische Sulfone und Sulfoxide, z.B. Dimethylsulfoxid und Sulfolan;

— aliphatische Nitrile, besonders Alkylnitrile mit 2-5 C-Atomen, wie Acetonitril, Propionitril und Butyronitril; 3-Alkoxypropionitrile mit 1 oder 2 C-Atomen im Alkoxyteil, wie 3-Methoxypropionitril und 3-Äthoxypropionitril; aromatische Nitrile, vor allem Benzonitril; Alkyl- und Alkoxyalkylester von aliphatischen Monocarbonsäuren mit insgesamt 2-6 C-Atomen, wie Ameisensäuremethyl- und -äthylester, Essigsäuremethyl-, -äthyl-, n-n-butyl- und -isobutylester; cyclische Äther, wie Tetrahydrofuran, Tetrahydropyran und Dioxan; Dialkyläther mit je 1-4 C-Atomen in den Alkylteilen, wie Diäthyläther, Di-n-propyläther und Diisopropyläther; N,N-Dialkylamide von aliphatischen Monocarbonsäuren mit 1-3 C-Atomen im Säureteil, wie N,N-Dimethylformamid, N,N-Dimethylacetamid, N,N-Diäthylacetamid und N,N-Dimethylmethoxyacetamid.

Bevorzugt sind polare Lösungsmittel, vor allem solche, die mit Wasser mischbar sind, wie aliphatische Alkohole, aliphatische Diole, Äthylenglykolmonoalkyläther und Diäthylenglykolmonoalkyläther,cyclische Amide, Amide der Phosphorsäure oder der phosphorigen Säure, Sulfoxide, Alkylnitrile, cyclische Äther und N,N-Dialkylamide von aliphatischen Monocarbonsäuren der oben erwähnten Art, besonders Methanol, Äthanol, Propanol, Isopropanol, Äthylenglykol und Diäthylenglykol, Äthylenglykolmonomethyl- und -monoäthyläther, Diäthylenglykolmonomethyl- und -monoäthyläther, N-Methyl-2-pyrrolidon, Phosphorsäure-tris (dimethylamid), Phosphorigsäure-tris (dimethylamid), Dimethylsulfoxid, Acetonitril, Tetrahydrofuran, Dioxan, N,N-Dimethylformamid und N,N-Dimethylacetamid.

Als optisch aktive Basen bzw. schwefligsaure Salze einer optisch aktiven Base können im erfindungsgemässen Verfahren an sich bekannte Verbindungen, insbesondere Amine, eingesetzt werden. Als Beispiele geeigneter optisch aktiver Basen seien erwähnt: S(+)-2-Amino-1-butanol, R(−)-2-Aminobutanol, L(+)-threo-2-Amino-1-phenyl-1,3-propandiol, (−)-Brucin, (+)-Chinidin, (−)-Chinin, (−)-Chinchonidin, (+)-Cinchonin, (+)-Dehydroabietylamin, (−)-Digitonin, (+)-Yohimbin, (−)-Nicotin und weitere Alkaloide mit optisch aktiven Zentren, (−)-Ephedrin, (+)-Ephedrin, (—)-N-Methylephedrin, R(+)-1-Naphtyl-1-äthylamin, S(—)-1-Naphtyl-1-äthylamin, S(−)-1-Phenyläthylamin, R(+)-1-Phenyläthyläthylamin, (+)-Pseudoephedrin, (−)-α-Phenyl-β-p-tolyläthylamin und (−)-bzw. (+)-threo-1-(p-Nitrophenyl)-2-N,N-dimethylaminopropan-1,3-diol. Es können auch Ester von Aminosäuren in optisch aktiver Form, wie der L-Alaninmethylester, L-Leucinäthylester, L-Phenylalanin-tert-butylester, L-Methioninmethylester und der L-Valinbenzylester sowie weitere Derivate optisch aktiver Aminosäuren verwendet werden.

Bervorzugt verwendet man als optisch aktive Base A R(−)-2-Amino-1-butanol, (−)-Ephedrin, (+)-Ephedrin, S-(−)-1-Phenyläthylamin, R(+)-1-Phenyläthylamin oder (+)-threo-1-(p-Nitrophenyl)-2-N,N-dimethylaminopropan-1,3-diol oder die schwefligsauren Salze dieser Basen.

Die optische aktive Base A bzw. das schwefligsaure Salz einer optisch aktiven Base A werden zweckmässig in einer Menge von 0,5 bis 1,5 Mol-Äquivalenten, bezogen auf das racemische Cyclobutanon der Formel II, eingesetzt.

Das schwefligsaure Salz der optisch aktiven Base A kann als solches eingesetzt oder in situ erzeugt werden. Vorzugsweise wird das schwefligsaure Salz der optisch aktiven Base A in situ erzeugt. Hierzu löst man das racemische Cyclobutanon der Formel II und die optisch aktive Base A in einem wasserhaltigen Lösungsmittel der vorgenannten Art und leitet Schwefeldioxid in die Lösung ein. Dabei werden Wasser und Schwefeldioxid in einer Menge von mindestens einem Mol-Äquivalent, bezogen auf die optisch aktive Base A verwendet.

Die Trennung von Gemischen diastereomerer Salze der Formel III kann nach an sich bekannten Methoden vorgenommen werden. In der Regel erfolgt die Trennung durch fraktionierte Kristallisation in einem geeigneten inerten organischen Lösungsmittel, besonders in mit Wasser mischbaren organischen Lösungsmitteln der oben erwähnten Art, vor allem in wässrigem Methanol, Äthanol, Acetonitril, Tetrahydrofuran und Dioxan.

Die diastereomeren Salze der Formel III sind neue Verbindungen und ebenfalls Gegenstand der Erfindung.

Bevorzugte Verbindungen der Formel III sind solche, worin A die oben angegebene bevorzugte Bedeutung hat, X Chlor oder Brom, eines von $R_1$ und $R_2$ Methyl und das andere Wasserstoff oder Methyl oder $R_1$ und $R_2$ zusammen Alkylen mit 2-3 C-Atomen, besonders Äthylen, und Y Chlor, Brom oder eine Gruppe $-OSO_2R'$ darstellen, worin R' Alkyl oder Halogenalkyl mit 1-4 C-Atomen, Benzyl, Phenyl, Methylphenyl, Nitrophenyl, Chlorphenyl oder Bromphenyl bedeutet, und vor allem Verbindungen der Formel II und III, worin A die oben erwähnte bevorzugte Bedeutung hat und X Chlor, $R_1$ und $R_2$ je Methyl und Y eine Gruppe $-OSO_2R'$ darstellen, worin R' Methyl, 4-Methylphenyl oder 4-Bromphenyl bedeutet.

Besonders bevorzugt sind Verbindungen der Formel III, worin A die oben genannte bevorzugte Bedeutung hat, X und Y unabhängig voneinander Chlor oder Brom, eines von $R_1$ und $R_2$ Methyl und das andere Wasserstoff oder Methyl oder $R_1$ und $R_2$ zusammen Alkylen mit 2-3 C-Atomen, besonders Äthylen, bedeuten, vor allem derartige Verbindungen, worin $R_1$ und $R_2$ je Methyl bedeuten. Ganz besonders bevorzugt sind Verbindungen der Formel III, worin A die oben genannte bevorzugte Bedeutung hat, $R_1$ und $R_2$ je Methyl und X und Y je Chlor oder X Brom und Y Chlor darstellen.

Ein diastereoisomeres Salz der Formel III kann in Gegenwart einer Base direkt in ein optisch aktives Cyclopropancarbonsäurederivat der Formel I übergeführt werden. Vorzugsweise wird jedoch aus dem reinen diastereomeren Salz der Formel III durch Zersetzung in Gegenwart einer Säure oder einer Base das optisch aktive Cyclobutanon der Formel II gewonnen und anschliessend in Gegenwart einer Base in ein optisch aktives Cyclopropancarbonsäurederivat der Formel I überführt.

Die Zersetzung der reinen Diastereomeren der Formel III zu einem optisch aktiven Cyclobutanon der Formel II wird ebenfalls zweckmässig in Gegenwart eines inerten organischen Lösungsmittels, besonders Äthanol oder Acetonitril, bei einer Temperatur von etwa 30 bis 70°C und vorzugsweise in Gegenwart eines Mol-Äquivalents einer anorganischen oder organischen Protonensäure durchgeführt. Bei der Zersetzung der Verbindungen der Formel III wird neben dem Cyclobutanon der Formel II auch die optisch aktive Base freigesetzt. Diese kann in sehr guter Ausbeute zurückgewonnen werden.

Beispiele geeigneter anorganischer Protonensäuren sind Halogenwasserstoffsäuren, wie HCl, HBr, HF und HJ, Salpetersäure, Phosphorsäure und Schwefelsäure. Als organische Protonensäuren kommen z.B. in Betracht: Sulfinsäuren, wie Benzolsulfinsäure; aliphatische und gegebenenfalls substituierte aromatische Sulfonsäuren, wie Methansulfonsäure, Benzolsulfonsäure, p-Toluolsulfonsäure, Naphthalinsulfonsäure, Naphthalin-1,5-disulfonsäure; aliphatische Monocarbonsäuren mit vorzugsweise 1-18 C-Atomen, wie Ameisensäure, Essigsäure, Propionsäure, Buttersäure, Laurinsäure, Palmitinsäure, Stearinsäure; halogenhaltige Monocarbonsäuren, wie Chloressigsäure, Dichloressigsäure, Trichloressigsäure und Trifluoressigsäure; aliphatische Dicarbonsäure mit vorzugsweise 2-12 C-Atomen, wie Malonsäure, Bernsteinsäure, Adipinsäure, Sebacinsäure; gegebenenfalls substituierte aromatische Mono- und Dicarbonsäuren, wie Benzoesäure, Toluylsäure, Naphthoesäure, Phthalsäure und Terephthalsäure; aliphatische und aromatische Phosphon- und Phosphinsäuren, wie Methyl-, Benzyl- oder Phenylphosphonsäure oder Dimethyl- und Diäthylphosphonsäure oder Diäthyl- und Benzolphosphinsäure.

Bevorzugt verwendet man als Protonensäure Salzsäure.

Eine andere Art der Freisetzung des optisch aktiven Cyclobutanons der Formel II besteht darin,

dass man die Salze der Formel III in Gegenwart von Wasser und einem organischen Lösungsmittel, vorzugsweise einem mit Wasser nicht mischbaren Lösungsmittel, wie gegebenenfalls chlorierte Kohlenwasserstoffe oder Dialkyläther, bei einem pH-Wert über 7,0 zersetzt. Zur Vermeidung der bei höheren pH-Werten auftretenden Favorski-Reaktion arbeitet man vorzugsweise bei pH-Werten zwischen 7,0 und 7,5 und bei Temperaturen unter 25°C. Bei einer besonders schonenden und zeitsparenden Variante dieser Zersetzungsweise wird die dabei freigesetzte schweflige Säure mit Hilfe eines schwachen Oxidationsmittels, wie einem Eisen-(III)-salz oder Jod, oxidiert. Die ebenfalls freigesetzte optisch aktive Base kann durch anschliessende Extraktion mit wässriger Säure vom Cyclobutanon getrennt und zurückgewonnen werden.

Die Überführung der reinen Diastereomeren der Formel III bzw. der optisch aktiven Cyclobutanone der Formel II in optisch aktive Cyclopropancarbonsäurederivate der Formel I erfolgt in Gegenwart einer Base. Als solche kommen beispielsweise Hydroxide bzw. Alkoholate der Formel IV

$$(IV) \qquad M^{n+}(O^{-}R)_n$$

in Betracht, worin M ein Alkalimetall- oder Erdalkalimetallkation und n die Zahl 1 oder 2 bedeuten und R die unter Formel I angegebene Bedeutung hat, wie Natrium-, Kalium-, Calcium- und Bariumhydroxid oder Natrium- und Kaliummethylat, -äthylat, -isopropylat, -sek-butylat, -tert-butylat, Magnesiummethylat oder Natrium- und Kaliumsalze des Benzylalkohols, des m-Phenoxybenzylalkohols des Furfurylalkohols oder des 2-Thiophenmethanols.

Geeignete Basen sind ferner Alkalimetall- und Erdalkalimetallcarbonate und Alkalimetallhydrogencarbonate, wie Calcium-, Barium-, Kalium- und Natriumcarbonat, Natrium- und Kaliumhydrogencarbonat. Unter Umständen kann die Reaktion auch durch Zusatz von geeigneten Halogenidakzeptoren, z.B. Silbernitrat, beschleunigt werden.

Die Basen werden in mindestens stöchiometrischer Menge, vorzugsweise jedoch im Überschuss eingesetzt.

Die Umsetzung wird je nach Art der Base zweckmässig in wässrigem, wässrig-organischem oder organischem Medium vorgenommen. Wird als Base ein Alkalimetall- oder Erdalkalimetallcarbonat verwendet, so wird die Umsetzung in wässrigem oder wässrig-organischem Medium durchgeführt. Auch die Umsetzung in Gegenwart von Alkalimetall- oder Erdalkalimetallhydroxiden und Alkalimetallhydrogencarbonaten wird mit Vorteil in wässrigem oder wässrig-organischem Medium vorgenommen. Dabei werden nach Ansäuren des Reaktionsgemisches, z.B. durch Zugabe von konzentrierter Salzsäure, Verbindungen mit R=H enthalten, die sich auf bekannte Weise in Derivate der Formel I R≠H übergeführt werden können, z.B. durch Überführung in das entsprechende Säurechlorid und Umsetzung mit einem vom Rest R abgeleiteten Alkohol.

Geeignete organische Lösungsmittel für die Umsetzung in wässrig-organischem oder organischem Medium sind niedere Alkohole, z.B. solche mit bis zu 6 C-Atomen, Benzylalkohol, aliphatische und cyclische Äther, wie Diäthyläther, Di-n-propyläther, Diisopropyläther, Tetrahydrofuran, Tetrahydropyran und Dioxan sowie aliphatische, cycloaliphatische oder aromatische Kohlenwasserstoffe, wie n-Pentan, n-Hexan, Cyclohexan, Benzol, Toluol und Xylole.

Die Favorski-Reaktion wird zur Bildung eines Cyclopropancarbonsäurederivats mit einem hohen Gehalt an cis-Isomeren vorteilhaft bei −10 bis +10°C durchgeführt. Anschliessend wird das Reaktionsgemisch zur vollständigen Überführung der Trihalogenäthylgruppe in eine Dihalogenvinylgruppe auf höhere Temperatur, gegebenenfalls bis zum Siedepunkt des Reaktionsmediums, erhitzt.

Bei der Umsetzung der reinen Diastereomeren der Formel III in Gegenwart einer Base entstehen intermediär optisch aktive Cyclobutanone der Formel II.

Nach einer bevorzugten Ausführungsform werden die reinen Diastereomeren der Formel III wie beschrieben zu optisch aktiven Cyclobutanonen der Formel II zersetzt, worauf man diese in Gegenwart einer Base in optisch aktive Verbindungen der Formel I überführt, wobei zur Hauptsache Verbindungen der Formel I in cis-Konfiguration entstehen. Anschliessend können die Verbindungen der Formel I gewünschtenfalls zu Verbindungen der Formel I' umgesetzt werden.

Die optisch aktiven Cyclobutanone der Formel II mit der unerwünschten Konfiguration können in Gegenwart eines Katalysators in entsprechende Racemate mit hohem Anteil an optisch aktivem Cyclobutanon mit der gewünschten Konfiguration überführt werden, die wiederum zur Gewinnung von diastereomeren sulfonsauren Salzen der Formel III und damit zur Gewinnung von optisch aktiven Cyclobutanonen der Formel II mit gewünschter Konfiguration verwendet werden können.

Als Katalysatoren für die Racemisierung der optisch aktiven Cyclobutanone der Formel II mit der unerwünschten Konfiguration eignen sich Säuren, quaternäre Ammoniumhalogenide oder Basen.

Als saure Katalysatoren können z.B. anorganische oder organische Protonensäuren der vorangehend genannten Art oder aber Lewis-Säuren, wie Bortrichlorid, Bortrifluorid, Aluminiumtrichlorid, Eisentrichlorid oder Zinkchlorid, verwendet werden.

Ferner können Salze von Protonensäuren, besonders Halogenwasserstoffsäuren, mit Ammoniak oder einer Stickstoff enthaltenden organischen Base eingesetzt werden. Als Stickstoff enthaltende organische Basen eignen sich aliphatische, cycloaliphatische, araliphatische und aromatische primäre, sekundäre und tertiäre Amine sowie heterocyclische Stickstoffbasen. Als Beispiele seien genannt: primäre aliphatische Amine mit bis zu 12 C-Atomen, wie Methylamin, Äthylamin, n-Butylamin, n-Octylamin, n-Dodecylamin,

Hexamethylendiamin, Cyclohexylamin, Benzylamin; sekundäre aliphatische Amine mit bis zu 12 C-Atomen, wie Dimethylamin, Diäthylamin, Di-n-propylamin, Dicyclohexylamin, Pyrrolidin, Piperidin, Piperazin, Morpholin; tertiäre aliphatische Amine, besonders Trialkylamine mit je 1-4 C-Atomen in den Alkylteilen, wie Triäthylamin, Tri-n-butylamin, N-Methylpyrrolidin, N-Methylmorpholin, 1,4-Diazabicyclo[2.2.2]octan, Chinuclidin; gegebenenfalls substituierte primäre, sekundäre und tertiäre aromatische Amine, wie Anilin, Toluidin, Naphthylamin, N-Methylanilin, Diphenylamin und N,N-Diäthylanilin; ferner Pyridin, Picolin, Indolin und Chinolin.

Bevorzugt sind Salze der Formel

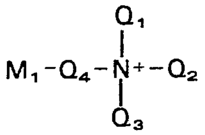

worin $M_1$ Fluor, Brom oder Jod, insbesondere Chlor, $Q_1$, $Q_2$ und $Q_3$ unabhängig voneinander Wasserstoff oder Alkyl mit 1-18 C-Atomen und $Q_4$ Wasserstoff, Alkyl mit 1-18 C-Atomen, Cyclohexyl, Benzyl, Phenyl oder Naphthyl bedeuten, sowie N-Alkyl-Pyridiniumhalogenide mit 1-18 C-Atomen im Alkyl, besonders die entsprechenden Chloride.

Beispiele derartiger Salze sind:
Ammoniumchlorid,
Ammoniumbromid,
Methylaminhydrochlorid,
Cyclohexylaminhydrochlorid,
Anilinhydrochlorid,
Dimethylaminhydrochlorid,
Diisobutylaminhydrochlorid,
Triäthylaminhydrochlorid,
Triäthylaminhydrobromid,
Tri-n-octylaminhydrochlorid,
Benzyldimethylaminhydrochlorid,
Tetramethyl-, Tetraäthyl-,
Tetra-n-propyl-,
Tetra-n-butylammoniumchlorid,
-bromid und -jodid,
Trimethylhexadecylammoniumchlorid,
Benzyldimethylhexadecylammoniumchlorid,
Benzyldimethyltetradecylammoniumchlorid,
Benzyltrimethyl-,
triäthyl- und -tri-n-butylammoniumchlorid,
n-Butyl-tri-n-propylammoniumbromid,
Octadecyltrimethylammoniumbromid,
Phenyltrimethylammoniumbromid oder -chlorid,
Hexadecylpyridiniumbromid und -chlorid.

Als basische Katalysatoren kommen organische Basen in Betracht, wie primäre, sekundäre und insbesondere tertiäre Amine der Formel

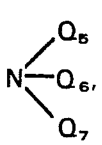
worin

$Q_5$ Alkyl mit 1-8 C-Atomen, Cycloalkyl mit 5 oder 6 C-Atomen, Benzyl oder Phenyl und $Q_6$ und $Q_7$ unabhängig voneinander Wasserstoff oder Alkyl mit 1-8 C-Atomen bedeuten, beispielsweise Triäthylamin, Tri-n-butylamin, Tri-isopentylamin, Tri-n-octylamin, N,N-Dimethylcyclohexylamin, N,N-Dimethylbenzylamin, N,N-Dimethyl-2-äthyl hexylamin, N,N-Diäthylanilin; ferner cyclische Amine, wie Pyridin, Chinolin, Lutidin, N-Alkylmorpholine, wie N-Methylmorpholin, N-Alkylpiperidine, wie N-Methyl- und N-Äthylpiperidin, N-Alkylpyrrolidine, wie N-Methyl- und N-Äthylpyrrolidin; Diamine, wie N,N,N',N'-Tetramethyläthylendiamin, N,N,N',N'-Tetramethyl-1,3-diaminobutan, N,N'-Dialkylpiperazine, wie N,N'-Dimethylpiperazin; bicyclische Diamine, wie 1,4-Diazabicyclo[2.2.2]octan und bicyclische Amidine, wie 1,5-Diazabicyclo[5.4.0]undec-5-en und 1,5-Diazabicyclo[4.3.0]non-5-en, und schliesslich polymere basische Verbindungen, wie p-Dimethylaminomethylpolystyrol.

Werden Säuren oder Basen im Überschuss eingesetzt, so können sie auch als Lösungsmittel dienen.

Als zusätzliche Co-Katalysatoren kann man Alkalimetallhalogenide, wie Kaliumjodid, Natriumjodid, Lithiumjodid, Kaliumbromid, Natriumbromid, Lithiumbromid, Kaliumchlorid, Natriumchlorid Lithiumchlorid, Kaliumfluorid, Natriumfluorid und Lithiumfluorid verwenden.

Die Menge des eingesetzten Katalysators kann innerhalb breiter Grenzen variieren. Im allgemeinen wird der Katalysator in einer Menge von etwa 5 bis 100 Gewichtsprozent, bezogen auf die optisch aktive Verbindung der Formel II, eingesetzt.

Die Racemisierung kann sowohl in der Schmelze als auch in einem inerten organischen Lösungsmittel vorgenommen werden. Die Reaktionstemperaturen für die Racemisierung in der Schmelze liegen im allgemeinen etwa zwischen 50 und 150°C, insbesondere etwa 80 und 130°C.

Für die Racemisierung in der Schmelze eignen sich als Katalysatoren vor allem die vorerwähnten Salze von Halogenwasserstoffsäuren mit Ammoniak oder organischen stickstoffhaltigen Basen, wie Trialkylaminhydrochloride und -bromide mit je 1-8 C-Atomen in den Alkylteilen, und ganz besonders Tetraalkylammoniumhalogenide, vor allem -chloride, -bromide und -jodide, mit je 1-18 C-Atomen in den Alkylteilen. Es können auch Basen der genannten Art eingesetzt werden, besonders Trialkylamine mit je 1-8 C-Atomen in den Alkylteilen, wie Triäthylamin, Tri-n-butylamin und Äthyldiisopropylamin.

Geeignete inerte organische Lösungsmittel sind z.B. solche der bei der Herstellung der Salze der Formel III genannten Art sowie aliphatische Monocarbonsäuren. Die Reaktionstemperaturen für die Racemisierung in einem inerten organischen Lösungsmittel liegen etwa zwischen 0 und 150°C, besonders etwa 80 und 130°C. Bevorzugte Lösungsmittel sind Essigsäure und Äthanol, wobei man als Katalysatoren mit Vorteil Halogenwasserstoffsäuren, besonders Bromwasserstoffsäure oder Chlorwasserstoffsäure, verwendet.

In polaren Lösungsmitteln, wie aliphatischen Alkoholen oder Diolen, cyclischen Amiden, Amiden der Kohlensäure, Amiden der phosphorigen Säure, der Phosphorsäure, der Phenylphosphonsäure oder von aliphatischen Phosphonsäuren, Amiden der Schwefelsäure oder von aliphatischen oder aromatischen Sulfonsäuren, N,N-Dialkylamiden von aliphatischen Monocarbonsäuren, Nitrilen oder Dialkylsulfoxiden der vorerwähnten Art läuft die Racemisierung beim Erhitzen auch ohne Zusatz von Säuren, quaternären Ammoniumhalogeniden oder Basen ab. In diesen Fällen wirkt das Lösungsmittel als Katalysator.

Cyclobutanone der Formel II, worin $R_1$ und $R_2$ die angegebene Bedeutung haben und X und Y unabhängig von einander Chlor oder Brom bedeuten, können, ausgehend von leicht zugänglichen Ausgangsmaterialien, auf einfache Weise und in guter Ausbeute nach einer neuartigen Synthese dadurch hergestellt werden, dass man eine Verbindung der Formel V

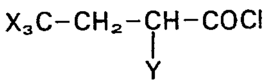 (V)

in Gegenwart einer organischen Base mit einem Olefin der Formel VI

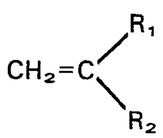 (VI)

zu einer Verbindung der Formel VII

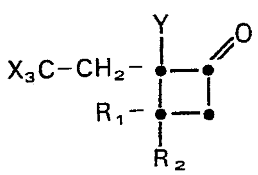 (VII)

umsetzt und die Verbindung der Formel VII in Gegenwart eines Katalysators zu einem Cyclobutanon der Formel II umlagert, wobei $R_1$, $R_2$, Y und X die angegebene Bedeutung haben (vergl. deutsche Offenlegungsschrift 2813337).

Cyclobutanone der Formel II, worin X, $R_1$ und $R_2$ die unter Formel I bzw. I' angegebene Bedeutung haben und Y eine Gruppe $-OSO_2R'$ darstellt, können dadurch erhalten werden, dass man eine Verbindung der Formel VII in Gegenwart einer anorganischen Base, wie Alkalimetall- oder Erdalkalimetallhydroxide, -carbonate oder -hydrogencarbonate, in ein 2,2,2-Trichlor- oder 2,2,2-Tribromäthylhydroxycyclobutanon der Formel VIIIa oder VIIIb

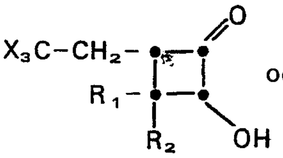 oder 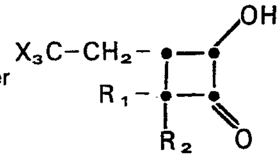

(VIIIa) (VIIIb)

überführt und die Verbindung der Formel VIIIa oder VIIIb anschliessend in Gegenwart einer organischen Base, wie tertiäre Amine, mit einer Verbindung $R'-SO_2-Cl$ umsetzt. Dabei erhält man im allgemeinen Gemische aus Cyclobutanonen der Formel II mit $Y=-OSO_2R'$ und Isomeren der Formel

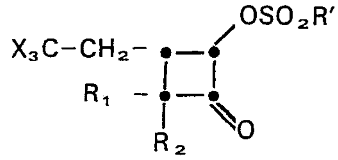

die auf übliche Weise voneinander getrennt werden können.

Bevorzugte Verbindungen der Formel I bzw. I' sind solche, in welchen R eine Alkylgruppe mit 1-4 Kohlenstoffatomen und vor allem Wasserstoff bedeutet.

Alkylgruppen R können geradkettig oder verzweigt sein. Als Beispiele geeigneter Alkylgruppen R seien genannt: die Methyl-, Athyl-, n-Propyl-, Isopropyl- und n-Butylgruppe. Bevorzugt sind die genannten Alkylgruppen geradkettig.

Alkylgruppen R' können ebenfalls geradkettig oder verzweigt sein und weisen vorzugsweise 1-4 C-Atome auf. Als Halogenalkylgruppen R' kommen insbesondere chlorierte, bromierte oder fluorierte, verzweigte oder geradkettige Alkylgruppen in Betracht. Als Beispiele geeigneter Halogenalkylgruppen R' seien genannt: die Chlormethyl-, Trifluormethyl- oder Perfluor-n-butylgruppe.

Bedeutet R' eine substituierte Phenylgruppe, so kommen als Substituenten vor allem Alkylgruppen mit 1-4 und insbesondere 1 oder 2 C-Atomen, Halogenatome, wie Chlor und Brom, und Nitrogruppen in Betracht. Bevorzugt weisen Phenylgruppen R' nur einen Substituenten der genannten Art auf. Beispiele geeigneter substituierter Phenylgruppen R' sind: die 3- oder 4-Methylphenyl-, 4-Äthylphenyl-, 2- und 4-Nitrophenyl-, 4-Chlor- und 4-Bromphenylgruppe.

Bedeutet R' eine Naphthylgruppe, so kann die Sulfonylgruppe in 1- oder 2-Stellung des Naphthalinrestes stehen.

Erfindungsgemäss erhaltene Verbindungen der Formel I oder I' mit R=H oder Alkyl mit 1-4 C-Atomen können auf an sich bekannte Weise in Verbindungen der Formel I oder I' übergeführt werden, worin R eine Gruppe

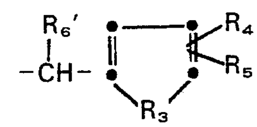 (b)

darstellt, $R_3$, $R_4$ und $R_5$ die unter Formel I bzw. I' angegebene Bedeutung haben und $R_6'$ Wasserstoff Äthinyl oder Cyano darstellt, z.B. durch Umsetzung mit entsprechenden Halogeniden oder Alkoholen (das letztere gegebenenfalls unter vorheriger Überführung der Verbindung der Formel I oder I' in das Säurechlorid) oder durch Umesterung.

Die Bromierung der Verbindungen der Formel I zu Verbindungen der Formel I' kann nach an sich bekannten Methoden, vor oder nach der allfälligen Überführung von Verbindungen der Formel I mit R=H oder Alkyl in Verbindungen der Formel I, worin R eine Gruppe (a) oder (b) darstellt, vorgenommen werden, z.B. nach der in der deutschen Offenlegungsschrift 2805312 beschriebenen Methode.

Bevorzugte Cyclobutanone der Formel II sind solche, worin X Chlor oder Brom, eines von $R_1$ und $R_2$ Methyl und das andere Wasserstoff oder Methyl oder $R_1$ und $R_2$ zusammen Alkylen mit 2-3 C-Atomen, besonders Äthylen, und Y Chlor, Brom oder eine Gruppe $-OSO_2R'$ darstellen, worin R' Alkyl oder Halogenalkyl mit 1-4 C-Atomen, Benzyl, Phenyl, Methylphenyl, Nitrophenyl, Chlorphenyl oder Bromphenyl bedeutet, und vor allem Verbindungen der Formel II und III, worin A die oben erwähnte bevorzugte Bedeutung hat und X Chlor, $R_1$ und $R_2$ je Methyl und Y eine Gruppe $-OSO_2R'$ darstellen, worin R' Methyl, 4-Methylphenyl oder 4-Bromphenyl bedeutet.

Besonders bevorzugt sind Cyclobutanone der Formel II, worin X und Y unabhängig voneinander Chlor oder Brom, eines von $R_1$ und $R_2$ Methyl und das andere Wasserstoff oder Methyl oder $R_1$ und $R_2$ zusammen Alkylen mit 2-3 C-Atomen, besonders Äthylen, bedeuten, vor allem derartige Verbindungen, worin $R_1$ und $R_2$ je Methyl bedeuten. Ganz besonders bevorzugt sind Cyclobutanone der Formel II, worin $R_1$ und $R_2$ je Methyl und X und Y je Chlor oder X Brom und Y Chlor darstellen.

Die Verbindungen der Formel I und I', worin R eine Gruppe

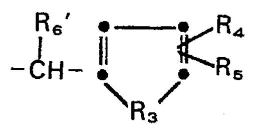

(b)

bedeutet und $R_3$, $R_4$, $R_5$ und $R_6'$ die angegebene Bedeutung haben, eignen sich zur Bekämpfung von verschiedenartigen tierischen oder pflanzlichen Schädlingen, insbesondere Insekten. Die Eigenschaften, Anwendungsgebiete und Verwendungsformen dieser Wirkstoffe sind in der Literatur beschrieben [vergl. z.B. „Nature", *246*, 169-170 (1973); „Nature", *248*, 710-711 (1974); „Proceedings 7th British Insecticide and Fungicide Conference", 721-728 (1973); „Proceedings 8th British Insecticide and Fungicide Conference", 373-378 (1975); „J. Agr. Food Chem.", *23*, 115 (1973); U.S. Patenschrift 3961070; deutsche Offenlegungsschriften 2553991, 2439177, 2326077 und 2614648].

Das erfindungsgemässe Verfahren ermöglicht auf einfache Weise die Herstellung von optisch aktiven Cyclopropancarbonsäurederivaten der Formel I bzw. I' mit cis-Konfiguration. Dies ist von besonderem Interesse, da die Cyclopropancarbonsäurederivate der Formel I bzw. I', in denen R eine Gruppe (b) bedeutet, eine wesentlich stärkere insektizide Wirkung besitzten als die entsprechenden Verbindungen mit trans-Konfiguration.

Ein besonderer Vorteil des erfindungsgemässen Verfahrens besteht darin, dass sich die dabei erhaltenen optisch aktiven Cyclobutanone der Formel II mit der unerwünschten Konfiguration in Gegenwart von Katalysatoren wieder in das Ausgangsracemat mit hohen Anteilen an cis-Konfiguration überführen lassen.

Das erfindungsgemässe Verfahren wird durch die folgenden Beispiele näher erläutert.

*Beispiel 1*

*Salz der 2-Chlor-3,3-dimethyl-1-hydroxy-4-(2',2',2'-trichloräthyl)-cyclobutan-1-sulfonsäure mit (−)-oder (+)-1-Phenyläthylamin.*

a) In eine Lösung von 105,6 g (0,4 Mol) racemischem 2-Chlor-3,3-dimethyl-4-(2',2',2-trichloräthyl)-cyclobutan-1-on und 48,5 g (0,4 Mol) (−)-1-Phenyläthylamin in 2 Liter Acetonitril und 40 ml Wasser wird während 90 Minuten bei Raumtemperatur (20-25°C) unter Rühren Schwefeldioxid eingeleitet. Das Reaktionsgemisch, das gemäss dünnschichtchromatographischer Kontrolle keine Ausgangsmaterialien mehr enthält, wird hierauf filtriert, und das Reaktionsprodukt, eine weisses Pulver, wird trockengesaugt; Ausbeute 203,1 g (>100% d. Th.). Anschliessend wird das Rohprodukt in der fünffachen (ml/g) Menge Äthanol/Wasser im Volumenverhältnis von 1:1 bei 60-70°C gelöst. Das nach dem Abkühlen erhaltene Produkt wird abfiltriert und trockengesaugt. Dieses Kristallisationsverfahren wird insgesamt 4× durchgeführt; Ausbeute 60,6 g feine weisse Nadeln; Smp. 136-138°C (Zersetzung). Durch Aufarbeiten der Mutterlaugen kann weiteres Produkt gewonnen werden.

b) In analoger Weise werden 26,4 g (0,1 Mol) racemisches 2-Chlor-3,3-dimethyl-4-(2',2',2'-trichloräthyl)-cyclobutan-1-on mit 12,1 g (+)-1-Phenyläthylamin in 500 ml Acetonitril und 10 ml Wasser mit Schwefeldioxid umgesetzt. Das Rohprodukt wird analog 1a) umkristallisiert; Smp. 134-138°C (Zersetzung).

Das als Ausgangsprodukt verwendete racemische 2-Chlor-3,3-dimethyl-4-(2',2',2'-trichloräthyl)-cyclobutan-1-on kann gemäss der deutschen Offenlegungsschrift 2813337 hergestellt werden.

*Beispiel 2*

*(+)-2-Chlor-3,3-dimethyl-4-(2',2',2'-trichloräthyl)-cyclobutan-1-on.*

a) 5,0 g des gemäss Beispiel 1a) erhaltenen Salzes werden in einer Lösung von 60 ml Äthanol und 25 ml 6N Salzsäure suspendiert. Man rührt 1 Stunde bei 60°C, lässt erkalten und engt anschliessend am Rotationsverdampfer auf ca. 1/3 des Volumens ein. Man verdünnt mit Wasser und nimmt das sich abscheidende Cyclobutanon in Diäthyläther auf. Nach dem Trocknen des organischen Extraktes über Natriumsulfat wird eingedampft. Beim Stehenlassen kristallisieren 2,6 g (92% d. Th.) Cyclobutanon aus, das folgende Drehwerte besitzt: $[\alpha]^{20}_{365} = +104°$, $[\alpha]^{20}_{436} = +22°$, $[\alpha]^{20}_{546} = +4°$, $[\alpha]^{20}_{578} = +2°$, $[\alpha]^{20}_{589} = +2°$ ($CCl_4$, 0,85%).

b) Eine Suspension von 10,7 g des nach Beispiel 1a) erhaltenen Salzes in 120 ml Wasser wird mit Äther überschichtet und unter intensivem Rühren durch Zugabe von wässriger Natriumbicarbonatlösung schwach alkalisch (pH 8-9) gestellt. Nach

Abtrennung der Ätherphase wird die wässrige Phase noch mehrmals mit Äther extrahiert. Die vereinigten Extrakte werden zuerst mit 6N-Salzsäure und dann mit Wasser gewaschen und über Magnesiumsulfat getrocknet. Nach Abdampfen des Äthers und Kristallisation des Rückstandes werden 4,9 g (+)-2-Chlor-3,3-dimethyl-4-(2',2',2'-trichloräthyl)-cyclobutan-1-on mit einem Drehwert $[\alpha]^{20}_{365}$=+105° ($CCl_4$, 0,82%) erhalten.

Aus dem salzsauren Waschwasser werden durch Zugabe von Natronlauge und Extraktion mit Äther 2,55 g (−)-1-Phenyläthylamin erhalten.

*Beispiel 3*

*(+)-cis-2-(2',2'-Dichlorvinyl)-3,3-dimethylcyclopropan-1-carbonsäure.*

a) 2,2 g (8,3 mMol) des gemäss Beispiel 2) erhaltenen (+)-Cyclobutanons werden zusammen mit 10 ml 2,5N Natronlauge während 8 Stunden bei 0°C sowie über Nacht bei Raumtemperatur gerührt. Danach wird die Reaktionslösung, die gemäss NMR-Spektrum ein Gemisch der cis- und der trans-2-(2',2',2-Trichloräthyl)-3,3-dimethylcyclopropan-1-carbonsäure im Verhältnis von ca. 80:20 enthält, während 2½ Stunden unter Rückfluss gekocht. Nach dem Erkalten säuert man an, das Produkt wird in Diäthyläther aufgenommen, und die Extrakte werden über Natriumsulfat getrocknet. Beim Eindampfen erhält man 1,5 g (86% d. Th.) 2-(2',2'-Dichlorvinyl)-3,3-dimethylcyclopropan-1-carbonsäure als 83:17cis/trans-Gemisch. Aus diesem wird die (+)-cis-Säure durch Chromatographie an Kieselgel in reiner Form abgetrennt. Drehung: $[\alpha]^{20}_{D}$ = +29° ($CHCl_3$, 1,1%).

b) 4,9 g (105 mMol) des gemäss Beispiel 1b) erhaltenen Salzes werden zu 42 ml auf 0°C gekühlte 1,25N Natronlauge gegeben, und die Suspension wird während 2 Stunden bei 0°C gerührt. Danach wird die Reaktionslösung, die gemäss NMR-Spektrum ein Gemisch der cis- und der trans-2-(2',2',2'-Trichloräthyl)-3,3-dimethylcyclopropan-1-carbonsäure im Verhältnis von ca. 55:45 enthält, während 3 Stunden unter Rückfluss erhitzt. Nach dem Erkalten wird die Reaktionslösung mehrmals mit Diäthyläther extrahiert, wobei das eingesetzte (+)-1-Phenyläthylamin zurückgewonnen wird. Die alkalische Wasserphase wird hierauf mit verdünnter Schwefelsäure angesäuert, und das Reaktionsprodukt wird in Diäthyläther aufgenommen. Nach dem Trocknen des Extraktes über Natriumsulfat wird eingeengt. Man erhält 2,0 g (91% d. Th.) 2-(2',2'-Dichlorvinyl)-3,3-dimethylcyclopropan-1-carbonsäure als ca. 60:40 cis/trans-Gemisch. Durch Chromatographie dieses Gemisches an Kieselgel (Elutionsmittel Hexan/Diäthyläther im Volumenverhältnis 1:1) wird zuerst die (−)-cis-Säure abgetrennt, $[\alpha]^{20}_{D}$ = −29° ($CHCl_3$, 1,3%).

Die späteren Fraktionen liefern die (+)-trans-Säure, die durch Umkristallisieren aus n-Hexan in reiner Form erhalten wird; $[\alpha]^{20}_{D}$ = +34° ($CHCl_3$, 1,3%).

*Beispiel 4*

*Salz der 2-Chlor-3,3-dimethyl-1-hydroxy-4-(2',2',2'-tribromäthyl)-cyclobutan-1-sulfonsäure mit (−)-1-Phenyläthylamin.*

In eine Lösung von 21,0 g (53 mMol) racemischem 2-Chlor-3,3-dimethyl-4-(2',2',2'-tribromäthyl)-cyclobutan-1-on und 6,4 g (53 mMol) (−)-1-Phenyläthylamin in 270 ml Acetonitril und 6 ml Wasser wird während 1 Stunde unter Rühren bei einer Temperatur unter 30°C Schwefeldioxid eingeleitet und anschliessend während 1½ Stunde bei Raumtemperatur nachgerührt. Die gebildeten Kristalle werden abfiltriert und trockengesaugt. Anschliessend wird analog der im Beispiel 1a) beschriebenen Weise umkristallisiert. Ausbeute 8,9 g feine weisse Nadeln; Smp. 126-128° (Zersetzung). Aus den Mutterlaugen kann weiteres Material gewonnen werden.

Das als Ausgangsprodukt verwendete racemische 2-Chlor-3,3-dimethyl-4-(2',2',2'-tribromäthyl)-cyclobutan-1-on kann wie folgt hergestellt werden:

324,8 g (1,0 Mol) 4,4,4-Tribrombuttersäure werden mit 600 g Thionylchlorid und 1 ml Dimethylformamid zunächst während 2 Stunden auf 40°C und dann während 3 Stunden auf 75°C erwärmt. Danach wird das überschüssige Thionylchlorid abdestilliert und der Rückstand im Hochvakuum rektifiziert. Es werden 326,0 g (95% der Theorie) 4,4,4-Tribrombuttersäurechlorid vom Siedepunkt 71 bis 73°C/0,05 Torr erhalten.

343,2 g (1,0 Mol) 4,4,4-Tribrombuttersäurechlorid werden in 600 g Thionylchlorid gelöst und bei 60°C unter gleichzeitiger Belichtung mit einer Quecksilberhochdrucklampe portionsweise mit 266,0 g (2,0 Mol) N-Chlorsuccinimid versetzt. Nach beendigter Zugabe des N-Chlorsuccinimids wird die erhaltene Mischung 5 Stunden bei 60°C unter Belichtung gerührt. Dann wird das Thionylchlorid abdestilliert und der Rückstand im Hochvakuum rektifiziert. Es werden 309,7 g (82% der Theorie) 2-Chlor-4,4,4-tribrombuttersäurechlorid vom Siedepunkt 59 bis 63°C/0,05 Torr erhalten.

In einem Autoklaven werden 90,6 g (0,24 Mol) 2-Chlor-4,4,4-tribrombuttersäurechlorid in 360 ml Cyclohexan vorgelegt und 134 g (2,4 Mol) Isobutylen aufgepresst. Anschliessend wird bei 65°C während 4 Stunden eine Lösung von 24,2 g (0,24 Mol) Triäthylamin in 120 ml Cyclohexan zugepumpt. Nach beendigter Zugabe der Triäthylaminlösung wird das Reaktionsgemisch noch 3 Stunden bei 65°C gehalten. Dann wird das gebildete Triäthylaminhydrochlorid abfiltriert und das Lösungsmittel abdestilliert. Der Rückstand wird in einem zu gleichen Teilen aus Toluol und Hexan bestehenden Lösungsmittelgemisch gelöst und an Kieselgel filtriert. Nach Abdampfen des Lösungsmittels werden aus dem Filtrat 51,4 g (54% der Theorie) 2-Chlor-3,3-dimethyl-2-(2',2',2'-tribromäthyl)-cyclobutan-1-on vom Schmelzpunkt 95 bis 97°C erhalten.

In 220 ml absolutes Äthanol werden nach Sättigung mit Chlorwasserstoff 22,8 g (0,054 Mol) 2-Chlor-3,3-dimethyl-2-(2',2',2'-tribromäthyl)-cyclobutan-1-on gelöst. Die erhaltene Lösung wird 5 Stunden bei 80°C gerührt. Dann wird das Reaktionsgemisch am Rotationsverdampfer auf etwa $^1/_3$ des Ausgangsvolumes eingeengt, mit Wasser versetzt und mit Äther extrahiert. Der Ätherextrakt wird zunächst mit gesättigter Kochsalzlösung, dann mit Natriumbicarbonatlösung gewaschen und über Natriumsulfat getrocknet. Der nach dem Abdampfen des Äthers erhaltene Rückstand wird an Kieselgel chromatographiert, wobei als Elutionsmittel Toluol dient. Nach Vereinigen und Eindampfen der reinen Fraktionen werden 17,1 g (75% der Theorie) 2-Chlor-3,3-dimethyl-4-(2',2',2'-tribromäthyl)-cyclobutan-1-on vom Schmelzpunkt 87 bis 89°C erhalten.

*Beispiel 5*

*(+)-2-Chlor-3,3-dimethyl-4-(2',2',2'-tribromäthyl)-cyclobutan-1-on.*

a) Aus 3,5 g (5,8 mMol) des gemäss Beispiel 4 erhaltenen Salzes werden auf die im Beispiel 2 beschriebene Weise 2,0 g (87% d. Th.) (+)-Cyclobutanon freigesetzt. Dieses besitzt folgende Drehwerte: $[\alpha]^{20}_{365}$ = +72°, $[\alpha]^{20}_{436}$ = +29°, $[\alpha]^{20}_{546}$ = +13°, $[\alpha]^{20}_{578}$ = +11°, $[\alpha]^{20}_{D}$ = +10°.

b) Eine Suspension von 10,6 g des gemäss Beispiel 4 erhaltenen Salzes in 250 ml Wasser wird mit 50 ml gesättigter Natriumbicarbonatlösung versetzt und mit Äther überschichtet. Dann wird unter intensivem Rühren eine durch Auflösen von 20 g Jod in 100 ml Äthanol hergestellte Jodlösung eingetropft bis die Färbung gerade erhalten bleibt (Verbrauch: ca. 20 ml). Die Ätherphase wird abgetrennt, zuerst mit Salzsäure dann mit Natriumthiosulfatlösung und anschliessend mit Sole gewaschen und über Natriumsulfat getrocknet. Nach Abdampfen des Äthers und Kristallisation des Rückstandes aus Hexan/Äther werden 6,2 g (+)-2-Chlor-3,3-dimethyl-4-(2',2',2'-tribromäthyl)-cyclobutan-1-on erhalten.

*Beispiel 6*

1,6 g (4 mMol) des gemäss Beispiel 5) erhaltenen Cyclobutanons werden zusammen mit 11 ml 1,25N Natronlauge während 22 Stunden bei 0°C gerührt. Danach wird die Reaktionslösung, die gemäss NMR-Spektrum ein Gemisch der cis- und der trans-2-(2',2',2'-Tribromäthyl)-3,3-dimethylcyclopropan-1-carbonsäure im Verhältnis von ca. 80:20 enthält, während 1 Stunde bei 80°C gerührt. Anschliessend wird wie im Beispiel 3 beschrieben verfahren. Man erhält 1,0 g (84%) 2-(2',2'-Dibromvinyl)-3,3-dimethylcyclopropan-1-carbonsäure als 80:20 cis/trans-Gemisch. Die (+)-cis-Säure wird durch Chromatographie an Kieselgel in reiner Form erhalten, $[\alpha]^{20}_{D}$ = +18°, Smp. 127-129°C.

*Beispiel 7*

a) 114,2 mg (−)-2-Chlor-3,3-dimethyl-4-(2',2',2'-trichloräthyl)-cyclobutan-1-on werden in 6 ml Essigsäure, die 16,5 Gew. % Bromwasserstoffsäure enthält, bei Raumtemperatur gelöst. Der am Anfang beobachtete Drehwert der Lösung von $[\alpha]^{20}_{546}$=−19,3° geht beim 20 stündigen Stehen auf $[\alpha]^{20}_{546}$=−0,6° zurück. Zur Aufarbeitung giesst man auf Wasser, extrahiert mehrmals mit n-Pentan, wäscht die Pentanextrakte mit verdünnter eiskalter Natriumbicarbonatlösung und trocknet über Natriumsulfat. Nach dem Eindampfen der Lösung erhält man 110 mg racemisches Cylobutanon, das gemäss NMR-Spektrum als 95:5 cis/trans-Gemisch vorliegt.

b) 264 mg (1 mMol) (−)-2-Chlor-3,3-dimethyl-4-(2',2',2'-trichloräthyl)-cyclobutan-1-on mit einem Drehwert von $[\alpha]^{20}_{365}$=−70° ($CCl_4$) werden zusammen mit 28 mg (0,1 mMol) Tetra-n-butylammoniumchlorid während 4 Stunden bei 120°C gerührt. Nach dem Abkühlen wird das Reaktionsprodukt mit Diäthyläther und Wasser versetzt. Die Ätherphase wird mit Wasser gewaschen, über Natriumsulfat getrocknet und über wenig Kieselgel filtriert. Nach dem Eindampfen des Filtrates erhält man 258 mg racemisches Cyclobutanon, das gemäss NMR-Spektrum als 90:10 cis/trans-Gemisch vorliegt.

*Beispiel 8*

*Auftrennung von 2-Chlor-3,3-dimethyl-4-(2',2',2'-trichloräthyl)-cyclobutan-1-on in die beiden optischen Antipoden.*

a) In eine Lösung von 10 g 2-Chlor-3,3-dimethyl-4-(2',2',2'-trichloräthyl)-cyclobutanon-1-on, 190 ml Acetonitril und 3,7 ml Wasser leitet man unter starkem Rühren 24 g $SO_2$ ein. Nun versetzt man die Lösung bei 20°C mit 6,2 g (+)-Ephedrin, gelöst in 30 ml Acetonitril, rührt eine Stunde, filtriert das Reaktionsgemisch und wäscht das Filtergut mit wenig Diäthyläther. Das gewonnene Salz wird zweimal aus je 45 ml 50%igem, wässerigem Äthanol umkristallisiert, dann zersetzt man es mit Salzsäure und erhält 2-Chlor-3,3-dimethyl-4-(2',2',2'-trichloräthyl)-cyclobutan-1-on mit einem Drehwert von $[\alpha]_{365}$=+105° (c=1,5, $CCl_4$).

b) Auf analoge Weise wie unter a) beschrieben, kann mit L-(+)-Threo-2-dimethylamino-1-(p-nitrophenyl)-1,3-propandiol optisch aktives 2-Chlor-3,3-dimethyl-4-(2',2',2'-trichloräthyl)-cyclo butan-1-on mit $[\alpha]_{365}$ = +107° (c = 0,9, $CCl_4$) hergestellt werden.

*Beispiel 9*

*Darstellung von (R)- und (S)-α-Cyano-3-phenoxybenzyl-(1R-cis)-2-(2',2'-dichlorvinyl)-3,3-dimethylcyclopropan-1-carboxylat (Isomer A und Isomer B).*

Zu einer Lösung von 1000 ml Methylenchlorid und 33,5 g N,N-Dimethylformamid tropft man bei −20°C nacheinander 19,6 g Oxalylchlorid, 16 g (1R-cis)-2-(2',2'-Dichlorvinyl)-3,3-dimethylcyclopropan-1-carbonsäure, gelöst in 100 ml $CH_2Cl_2$, 37,2 g Pyridin, gelöst in 50 ml $CH_2Cl_2$ und 17 g (R,S)-α-Cyano-3-phenoxybenzylalkohol, gelöst in 100 ml $CH_2Cl_2$. Das Reaktionsgemisch lässt man langsam auf Raumtemperatur erwärmen, rührt 16 Stunden bei 20°C, dampft unter vermindertem Druck zur Trockene ein, versetzt mit n-Hexan und wäscht die organische Phase mit

1N Salzsäure, gesättigter Natriumbicarbonatlösung und Sole, trocknet über $MgSO_4$ und destilliert das Lösungsmittel unter vermindertem Druck ab. Man erhält 30 g eines 1:1 Gemisches der Isomeren A und B. Durch Chromatographie an Kieselgel mit einem Gemisch von Petroläther-Diisopropyläther als Eluierungsmittel erhält man zuerst reines (R)-α-Cyano-3-phenoxybenzyl-(1R-cis)-2-(2′,2′-dichlorvinyl)-3,3-dimethylcyclopropan-1-carboxylat (Isomer A) und anschliessend (S)-α-Cyano-3-phenoxybenzyl-(1R-cis)-2-(2′,2′-dichlorvinyl)-3,3-dimethylcyclopropan-1-carboxylat (Isomer B).

*Isomer A:* $[\alpha]_D = -32°$ (c = 1,1 %, Benzol).
$n_D^{21} = 1,5665$;
*Isomer B:* $[\alpha]_D = +67°$ (c = 1,2%, Benzol).
Smp. = 55–56°C.

*Beispiel 10*

*(S)-α-Cyano-3-phenoxybenzyl-(1R-cis)-2-2′,2′-dichlor-(S resp. R)-1′,2′-dibromäthyl)-3,3-dimethyl-cyclopropan-1-carboxylat (Isomer 1 resp. Isomer 2).*

2 g (S)-α-Cyano-3-phenoxybenzyl-(1R-cis)-2-(2′,2′-dichlorvinyl)-3,3-dimethylcyclopropan-1-carboxylat werden in 30 ml $CCl_4$ gelöst und anschliessend portionenweise mit einer Lösung von 0,78 g Brom in 5 ml $CCl_4$ versetzt. Während der Zugabe des Broms wird das Reaktionsgefäss mit einer 6 Watt Tageslichtlampe bestrahlt. Nach 30 Minuten wird die Reaktionslösung unter vermindertem Druck zur Trockene eingeengt. Das Rohprodukt chromatographiert man an Kieselgel und eluiert mit einem Gemisch von n-Hexan und Diisopropyläther (10:1) zuerst das Isomer 1 mit $n_D^{21}$ = 1,5791 und dann das Isomer 2 mit $n_D^{21}$ = 1,5782.

*Beispiel 11*

*Racemisierung von (+)-2-Chlor-3,3-dimethyl-4-(2′,2′,2′-tribromäthyl)-cyclobutan-1-on.*

4,0 g (+)-2-Chlor-3,3-dimethyl-4-(2′,2′,2′-tribromäthyl)-cyclobutan-1-on ($[\alpha]_{365}^{20}$=72°) und 0,45 g Tetrabutylammoniumchlorid werden während 4 Stunden bei 110°C gerührt. Die abgekühlte Schmelze wird mit Äther digeriert. Die abgetrennte Ätherphase wird mit Wasser gewaschen und über wenig Kieselgel filtriert. Nach dem Abdampfen des Äthers erhält man 3,75 g racemisches 2-Chlor-3,3-dimethyl-4-(2′,2′,2′-tribromäthyl)-cyclobutan-1-on, das nach NMR-Spektrum als 95:5 cis/trans-Gemisch vorliegt.

Nach Umkristallisation aus Hexan/Äther werden 3,05 g racemisches cis-2-Chlor-3,3-dimethyl-4-(2′,2′,2′-tribromäthyl)-cyclobutan-1-on vom Smp. 88-89°C erhalten.

*Beispiel 12*

*a) Herstellung von (R,S)-α-Cyano-3-(4-fluorphenoxy)-benzyl-(1R-cis)-2-(2′,2′-dichlorvinyl)-3,3-dimethylcyclopropan-1-carboxylat (Isomer A und Isomer B).*

In einer Lösung von 67,7 g (1R-cis)-2-(2′,2′-Dichlorvinyl)-2,2-dimethylcyclopropan-1-carbonsäurechlorid in 500 ml Toluol werden bei 0-5°C unter Rühren zunächst 31,2 g Pyridin und anschliessend eine Lösung von 72,3 g (R,S)-α-Cyano-3-(4-fluorphenoxy)-benzylalkohol in 200 ml Toluol eingetropft. Danach wird das Kühlbad entfernt und 16 Stunden bei Raumtemperatur nachgerührt. Anschliessend wird das Reaktionsgemisch durch Zugabe von Wasser auf 1000 ml verdünnt und die Phasen getrennt. Die organische Phase wird zunächst zweimal mit je 500 ml 2N Salzsäure und dann nacheinander mit 1000 ml 10%iger Kaliumcarbonatlösung, 1000 ml gesättigter Natriumbicarbonatlösung und dreimal mit 1000 ml Sole gewaschen und über Magnesiumsulfat getrocknet. Durch Abdampfen des Lösungsmittels unter vermindertem Druck und Filtration des erhaltenen Rohprodukts an Kieselgel mit einem Gemisch aus 1 Teil Äther und 10 Teilen Hexan wird ein 1:1 Gemisch von (R)-α-Cyano-3-(4-fluorphenoxy)-benzyl-(1R-cis)-2-(2′,2′-dichlorvinyl)-3,3-dimethylcyclopropan-1-carboxylat (Isomer A) und (S)-α-Cyano-3-(4-fluorphenoxy)-benzyl-(1R-cis)-2-(2′,2′-dichlorvinyl)-3,3-dimethylcyclopropan-1-carboxylat (Isomer B) mit einem Brechungsindex von $n_D^{20}$ = 1,5531 erhalten.

Das Gemisch der Isomeren A und B wird durch Chromatographie an einer Silicagelsäule mit einem Gemisch von 85 Teilen Petroläther und 15 Teilen Diisopropyläther als Laufmittel aufgetrennt. Dabei wird zunächst Isomer A und dann Isomer B erhalten. Für die beiden Isomeren wurden folgende Drehwerte und Brechungsindizes gemessen:

*Isomer A:* $[\alpha]_D = -34° \pm 1°$ (c=1,291; Benzol)
$[\alpha]_D = -22° \pm 1°$ (c=1,407; Chloroform)
$n_D^{21}$ = 1,5580

*Isomer B:* $[\alpha]_D = +67° \pm 1°$ (c=1,277; Benzol)
$[\alpha]_D = +30° \pm 1°$ (c=1,069; Chloroform)
Smp. 59-60°C

Das Isomer B kann aus dem erhaltenen Gemisch der Isomeren A und B auch wie folgt abgetrennt werden:

Eine Lösung von 11 g des Gemisches der Isomeren A und B und 1,28 g Triäthylamin in 43 ml Isopropanol wird 24 Stunden bei 0°C gerührt. Der hierbei gebildete weisse Niederschlag wird abfiltriert und mit 5 ml eiskaltem Isopropanol gewaschen. Man erhält so Isomer B vom Smp. 59-60°C. Die in Benzol und Chloroform gemessenen Drehwerte sind identisch mit den Drehwerten des chromatographisch abgetrennten Isomeren B.

*b) Herstellung von (S)-α-Cyano-3-(4-fluorphenoxy)-benzyl-(1R-cis)-2-(1′,2′-dibrom-2′,2′-dichloräthyl)-3,3-dimethylcyclopropan-1-carboxylat.*

In einer Lösung von 22,4 g Isomer B und 41 mg α,α′-Azoisobutyronitril in 150 ml Kohlenstofftetrachlorid wird bei 60°C eine Lösung von 8,2 g Brom

in 10 ml Kohlenstofftetrachlorid eingetropft. Nach beendigter Zugabe der Bromlösung wird das Reaktionsgemisch 2½ Stunden auf Rückflusstemperatur erhitzt. Dann wird das Lösungsmittel unter vermindertem Druck abdestilliert. Das erhaltene Rohprodukt wird an Kieselgel mit einem Gemisch aus 95 Teilen Hexan und 5 Teilen Tetrahydrofuran chromatographiert. Man erhält so das (S)-α-Cyano-3-(4-fluorphenoxy)-benzyl-(1R-cis)-2-(1',2'-dibrom-2',2'-dichloräthyl)-3,3-dimethyl-cyclopropan-1-carboxylat mit einem Brechungsindex $n_D^{20}$ = 1,5680 und einem Drehwert von
$[\alpha]_D^{20}$ = +38° ±1° (c = 0,756; Benzol)
$[\alpha]_D^{20}$ = +21° ±1° (c = 1,066; Chloroform).

## Patentansprüche

1. Verfahren zur Herstellung von optisch aktiven Cyclopropancarbonsäurederivaten der Formel I oder I'

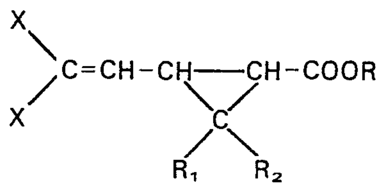
(I)

oder

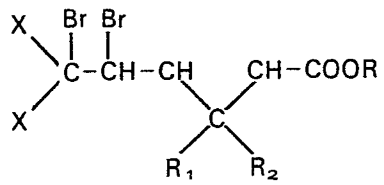
(I')

worin X Chlor oder Brom, einer der Reste $R_1$ und $R_2$ Methyl und der andere Wasserstoff oder Methyl oder $R_1$ und $R_2$ zusammen Alkylen mit 2-4 Kohlenstoffatomen und R Wasserstoff, Alkyl mit 1-4 Kohlenstoffatomen oder eine Gruppe a)

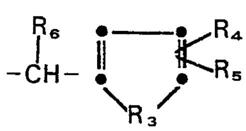
(a)

bedeutet, wobei $R_3$ Sauerstoff, Schwefel oder Vinylen, $R_4$ Wasserstoff, Methyl, Benzylphenoxy, 4-Methylphenoxy, 4-Chlorphenoxy, 4-Fluorphenoxy oder Phenylmercapto, $R_5$ Wasserstoff, Fluor, Chlor oder Methyl und $R_6$ Wasserstoff, Cyano oder Äthinyl bedeuten, dadurch gekennzeichnet, dass man ein Racemat eines Cyclobutanons der Formel II

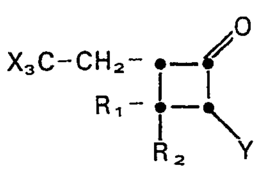
(II)

in welcher X, $R_1$ und $R_2$ die unter Formel I angegebene Bedeutung haben und Y Chlor, Brom oder eine Gruppe $-OSO_2R'$ bedeutet, wobei R' Alkyl, Halogenalkyl, Benzyl, Naphthyl oder gegebenenfalls substituiertes Phenyl darstellt, durch Umsetzung mit einem schwefeligsauren Salz einer optisch aktiven Base A in ein Gemisch von diastereomeren sulfonsauren Salzen der Formel III

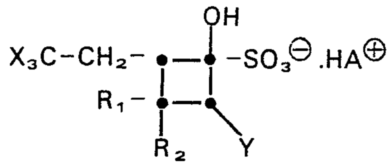
(III)

in welcher X, $R_1$, $R_2$ und Y die oben angegebene Bedeutung haben und A für eine optisch aktive Base steht, überführt, dieses Gemisch in die reinen diastereomeren sulfonsauren Salze der Formel III auftrennt und entweder direkt die reinen diastereomeren sulfonsauren Salze der Formel III oder die durch Zersetzung aus diesen erhaltenen optisch aktiven Cyclobutanone der Formel II in Gegenwart einer Base in ein optisch aktives Cyclopropancarbonsäurederivat der Formel I umwandelt und dieses gegebenenfalls durch Bromierung in ein optisch aktives Cyclopropancarbonsäurederivat der Formel I' überführt.

2. Ein Verfahren nach Anspruch 1, dadurch gekennzeichnet, dass man als optisch aktive Base R(−)-2-Amino-1-butanol, (−)-Ephedrin, (+)-Ephedrin, S(−)-1-Phenyläthylamin, R(+)-1-Phenyläthylamin oder (+)-Threo-1-(p-nitrophenyl)-2-N,N-dimethylaminopropan-1,3-diol oder ein schwefligsaures Salz dieser Basen verwendet.

3. Ein Verfahren nach einem der Ansprüche 1-2, dadurch gekennzeichnet, dass man ein Racemat einer Verbindung der Formel II verwendet, worin X und Y unabhängig voneinander Chlor oder Brom, eines von $R_1$ und $R_2$ Methyl und das andere Wasserstoff oder Methyl oder $R_1$ und $R_2$ zusammen Alkylen mit 2-3 C-Atomen bedeuten.

4. Ein Verfahren nach einem der Ansprüche 1-3, dadurch gekennzeichnet, dass man ein Racemat einer Verbindung der Formel II verwendet, worin X und Y je Chlor oder X Brom und Y Chlor und $R_1$ und $R_2$ je Methyl darstellen.

5. Ein Verfahren nach einem der Ansprüche 1-4, dadurch gekennzeichnet, dass man die Umsetzung des Racemats eines Cyclobutanons der Formel II bei einer Temperatur zwischen etwa 0 und 100°C in Gegenwart eines inerten organischen Lösungsmittels und in Gegenwart von Wasser und Schwefeldioxid vornimmt, wobei das Wasser und das Schwefeldioxid in einer Menge von mindestens 1 Mol-Äquivalent, bezogen auf die optisch aktive Base, verwendet werden.

6. Ein Verfahren nach einem der Ansprüche 1-5, dadurch gekennzeichnet, dass man die Auftrennung eines Gemisches diastereomerer Salze der Formel III durch fraktionierte Kristallisation in Gegenwart eines mit Wasser mischbaren inerten organischen Lösungsmittels vornimmt.

7. Ein Verfahren nach einem der Ansprüche 1-6, dadurch gekennzeichnet, dass man die Zersetzung des reinen Diastereomeren der Formel III zu einem optisch aktiven Cyclobutanon der Formel III bei einer Temperatur von etwa 30 bis 70°C in Gegenwart eines inerten organischen Lösungsmittels und in Gegenwart eines Mol-Äquivalents einer anorganischen oder organischen Protonensäure durchführt.

8. Ein Verfahren nach Anspruch 1, dadurch gekennzeichnet, dass man ein Racemat eines Cyclobutanons der Formel II, in welcher X und Y je Chlor oder X Brom und Y Chlor und $R_1$ und $R_2$ je Methyl bedeuten, in einem polaren, mit Wasser mischbaren Lösungsmittel aus der Gruppe Methanol, Äthanol, Acetonitril, Tetrahydrofuran und Dioxan bei 20-60°C in Gegenwart von mindestens einem Mol-Äquivalent Wasser und mindestens einem Mol-Äquivalent Schwefeldioxid mit einer optisch aktiven Base aus der Gruppe R-(−)-2-Amino-1-butanol, (−)-Ephedrin, (+)-Ephedrin, S-(−)-1-Phenyläthylamin, R-(+)-1-Phenyläthylamin und (+)-Threo-1-(p-nitrophenyl)-2-N,N-dimethylaminopropan-1,3-diol zu einem Gemisch von diastereomeren sulfonsauren Salzen umsetzt, dieses Gemisch durch fraktionierte Kristallisation trennt, das gewünschte diastereomere Salz in Äthanol oder Acetonitril in Gegenwart von Salzsäure zu dem optisch aktiven Cyclobutanon spaltet und dieses anschliessend in Gegenwart einer Base bei −10 bis +10°C in das entsprechende 2-(2',2',2'-Trihalogenäthyl)-cyclopropancarbonsäurederivat überführt und aus diesem durch Erhitzen Halogenwasserstoff abspaltet.

9. Eine Verbindung der Formel III

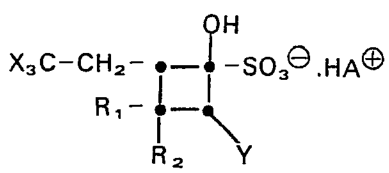

(III)

worin X Chlor oder Brom, eines von $R_1$ und $R_2$ Methyl und das andere Wasserstoff oder Methyl oder $R_1$ und $R_2$ zusammen Alkylen mit 2-4 C-Atomen, A eine optisch aktive Base und Y Chlor, Brom oder eine Gruppe $-OSO_2R'$ darstellen, worin R' Alkyl, Halogenalkyl, Benzyl, Naphthyl oder gegebenenfalls substituiertes Phenyl bedeutet.

10. Eine Verbindung der Formel III nach Anspruch 9, worin A R(−)-2-Amino-1-butanol, (−)-Ephedrin, (+)-Ephedrin, S(−)-1-Phenyläthylamin oder R(+)-1-Phenyläthylamin, X und Y unabhängig voneinander Chlor oder Brom, eines von $R_1$ und $R_2$ Methyl und das andere Wasserstoff oder Methyl oder $R_1$ und $R_2$ zusammen Alkylen mit 2-3 C-Atomen bedeuten.

11. Eine Verbindung der Formel III nach Anspruch 10, worin X und Y je Chlor oder X Brom und Y Chlor und $R_1$ und $R_2$ je Methyl bedeuten.

12. Ein Verfahren zur Herstellung einer Verbindung der Formel III nach Anspruch 9, dadurch gekennzeichnet, dass man ein Racemat eines Cyclobutanons der Formel II

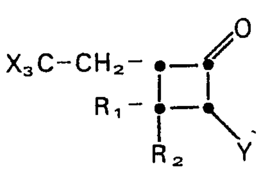

(II)

entwender mit dem schwefligsauren Salz einer optisch aktiven Base A oder in Gegenwart von Wasser und Schwefeldioxid mit einer optisch aktiven Base A umsetzt, wobei X, Y, $R_1$, $R_2$ und A die unter Formel III angegebene Bedeutung haben.

**Claims:**

1. A Process for the preparation of optically active cyclopropancarboxylic acid derivatives of the formula I or I'

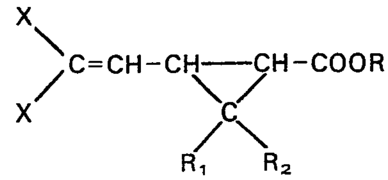

(I)

or

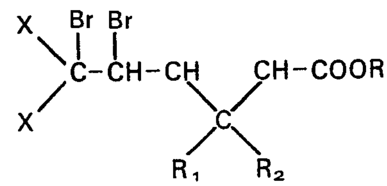

(I')

in which X is chlorine or bromine, one of the radicals $R_1$ and $R_2$ is methyl and the other is hydrogen or methyl, or $R_1$ and $R_2$ together are alkylene having 2-4 carbon atoms, and R is hydrogen, alkyl having 1-4 carbon atoms or a group a)

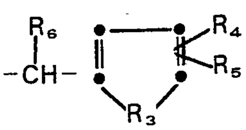

(a)

in which $R_3$ is oxygen, sulfur or vinylene, $R_4$ is hydrogen, methyl, benzyl, phenoxy, 4-methylphenoxy, 4-chlorophenoxy, 4-fluorophenoxy or phenylmercapto, $R_5$ is hydrogen, fluorine, chlorine or methyl and $R_6$ is hydrogen, cyano or ethynyl, which comprises converting a racemate of a cyclobutanone of the formula II

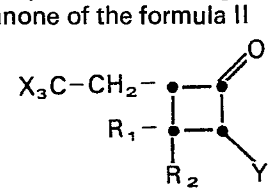

(II)

in which X, $R_1$ and $R_2$ are as defined under formula I and Y is chlorine, bromine or a group $-OSO_2R'$, in which R' is alkyl, halogenoalkyl, benzyl, naphthyl or substituted or unsubstituted phenyl, by reaction with a sulfurous acid salt of an optically active base A, to a mixture of diastereomeric sulfonic acid salts of the formula III

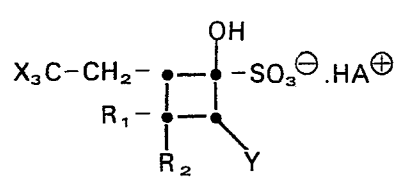

(III)

in which X, $R_1$, $R_2$ and Y are as defined and A is an optically active base, separating this mixture into the pure diastereomeric sulfonic acid salts of the formula III and converting either the pure diastereomeric sulfonic acid salts of the formula III direct, or the optically active cyclobutanones of the formula II obtained from the said salts by decomposition, in the presence of a base to an optically active cyclopropanecarboxylic acid derivative of the formula I and, if desired, converting the latter by bromination to an optically active cyclopropanecarboxylic acid derivative of the formula I'.

2. A process according to claim 1, wherein the optically active base used is R(−)-2-amino-1-butanol, (−)-ephedrine, (+)-ephedrine, S(−)-1-phenylethylamine, R(+)-1-phenylethylamine or (+)-threo-1-(p-nitrophenyl)-2-N,N-dimethylaminopropane-1,3-diol or a sulfurous acid salt of these bases.

3. A process according to either of claims 1 and 2, wherein a racemate of a compound of the formula II is used in which X and Y independently of one another are chlorine or bromine and one of $R_1$ and $R_2$ is methyl and the other is hydrogen or methyl, or $R_1$ and $R_2$ together are alkylene having 2-3 C atoms.

4. A process according to either of claims 1 to 3, wherein a racemate of a compound of the formula II is used in which X and Y are each chlorine, or X is bromine and Y is chlorine, and $R_1$ and $R_2$ are each methyl.

5. A process according to any one of claims 1 to 4, wherein the reaction of the racemate of a cyclobutanone of the formula II is carried out at a temperature between about 0 and 100°C in the presence of an inert organic solvent and in the presence of water and sulfur dioxide, the water and the sulfur dioxide being used in an amount of at least 1 mol equivalent, based on the optically active base.

6. A process according to any one of claims 1 to 5, wherein the separation of a mixture of diastereomeric salts of the formula III is effected by fractional crystallisation in the presence of a water-miscible inert organic solvent.

7. A process according to any one of claims 1 to 6, wherein the decomposition of the pure diastereomer of the formula III to an optically active cyclobutanone of the formula II is carried out at a temperature of about 30 to 70°C in the presence of an inert organic solvent and in the presence of one mol equivalent of an inorganic or organic proton acid.

8. A process according to claim 1, which comprises reacting a racemate of a cyclobutanone of the formula II, in which X and Y are each chlorine, or X is bromine and Y is chlorine, and $R_1$ and $R_2$ are each methyl, in a polar, water-miscible solvent from the group comprising methanol, ethanol, acetonitrile, tetrahydrofuran and dioxan, at 20 to 60°C in the presence of at least one mol equivalent of water and at least one mol equivalent of sulfur dioxide, with an optically active base from the group comprising R-(−)-2-amino-1-butanol, (−)-ephedrine, (+)-ephedrine, S-(−)-1-phenylethylamine, R-(+)-1-phenylethylamine and (+)-threo-1-(p-nitrophenyl) -2-N,N-dimethylaminopropane-1,3-diol, to give a mixture of diastereomeric sulfonic acid salts, separating this mixture by fractional crystallisation, splitting the desired diastereomeric salt in ethanol or acetonitrile in the presence of hydrochloric acid to give the optically active cyclobutanone and then converting the latter in the presence of a base at −10 to +10°C to the corresponding 2-(2',2',2'-trihalogenoethyl)-cyclopropanecarboxylic acid derivative and eliminating hydrogen halide from the latter by heating.

9. A compound of the formula III

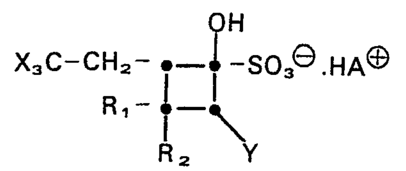

(III)

in which X is chlorine or bromine, one of $R_1$ and $R_2$ is methyl and the other is hydrogen or methyl, or $R_1$ and $R_2$ together are alkylene having 2-4 C atoms, A is an optically active base Y is chlorine, bromine or a group $-OSO_2R'$, in which R' is alkyl, halogenoalkyl, benzyl, naphthyl or substituted or unsubstituted phenyl.

10. A compound of the formula III according to claim 9, in which A is R(−)-2-amino-1-butanol, (−)-ephedrine, (+)-ephedrine, S(−)-1-phenylethylamine or R(+)-1-phenylethylamine, X and Y independently of one another are chlorine or bromine and one of $R_1$ and $R_2$ is methyl and the other is hydrogen or methyl, or $R_1$ and $R_2$ together are alkylene having 2-3 C atoms.

11. A compound of the formula III according to claim 10, in which X and Y are each chlorine, or X is bromine and Y is chlorine, and $R_1$ and $R_2$ are each methyl.

12. A process for the preparation of a compound of the formula III according to claim 9, which comprises reacting a racemate of a cyclobutanone of the formula II

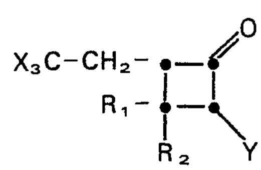

(II)

either with the sulfurous acid salt of an optically active base A or, in the presence of water and sulfur dioxide, with an optically active base A, X, Y, $R_1$, $R_2$ and A being as defined under formula III.

## Revendications

1. Procédé de préparation de dérivés d'acides cyclopropanecarboxyliques de formule I ou I' à l'état d'isomères optiques

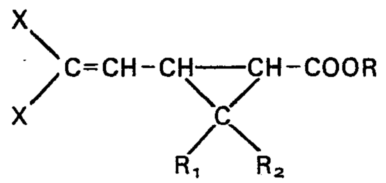

(I)

ou

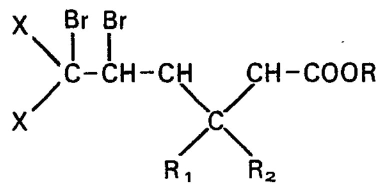

(I')

dans lesquelles X représente le chlore ou le brome, l'un des symboles $R_1$ et $R_2$ représente un groupe méthyle et l'autre l'hydrogène ou un groupe méthyle ou bien $R_1$ et $R_2$ forment ensemble un groupe alkylène en C2-C4 et R représente l'hydrogène, un groupe alkyle en C1-C4 ou un groupe a)

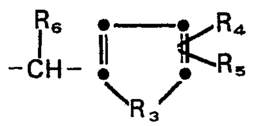

(a)

$R_3$ représente l'oxygène, le soufre ou un groupe vinylène, $R_4$ représente l'hydrogène, un groupe méthyle, benzyle, phénoxy, 4-méthylphénoxy, 4-chlorophénoxy, 4-fluorophénoxy ou phénylmercapto, $R_5$ représente l'hydrogène, le fluor, le chlore ou un groupe méthyle et $R_6$ représente l'hydrogène, un groupe cyano ou éthynyle, caractérisé en ce que l'on convertit un racémate d'une cyclobutanone de formule II

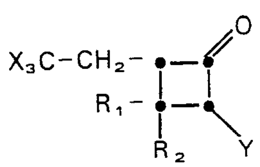

(II)

dans laquelle X, $R_1$ et $R_2$ ont les significations indiquées en référence à la formule I et Y représente le chlore, le brome ou un groupe $-OSO_2R'$, R' représentant un groupe alkyle, halogénoalkyle, benzyle, naphtyle ou phényle éventuellement substitué, par réaction avec un sulfite d'une base énantiomère A, en un mélange de sulfonates diastéréo-isomères de formule III

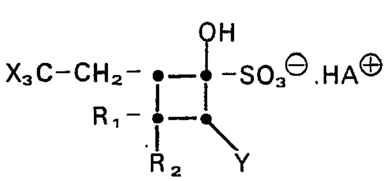

(III)

dans laquelle X, $R_1$, $R_2$ et Y ont les significations indiquées ci-dessus et A représente une base énantiomère, on sépare ce mélange en les sulfonates diastéréo-isomères purs de formule III qu'on convertit directement en un dérivé d'acide cyclopropanecarboxylique énantiomère de formule I ou bien on convertit les cyclobutanones de formule II à l'état d'énantiomères obtenus par décomposition de ces sulfonates en un dérivé d'acide cyclopropanecarboxylique énantiomère en présence d'une base, et on convertit le cas échéant le dérivé d'acide cyclopropanecarboxylique énantiomère par bromation en un dérivé d'acide cyclopropanecarboxylique énantiomère de formule I'.

2. Procédé selon la revendication 1, caractérisé en ce que l'on utilise en tant que base énantiomère le R(−)-2-amino-1-butanol, la (−)-éphédrine, la (+)-éphédrine, la S(−)-1-phényléthylamine, la R(+)-1-phényléthylamine ou le (+)-thréo-1-(p-nitrophényl)-2-N,N-diméthylaminopropane-1,3-diol ou un sulfite de ces bases.

3. Procédé selon l'une des revendications 1 et 2, caractérisé en ce que l'on utilise un racémate d'un composé de formule II dans laquelle X et Y représentent chacun, indépendamment l'un de l'autre, le chlore ou le brome, l'un des symboles $R_1$ et $R_2$ représente le groupe méthyle et l'autre l'hydrogène ou un groupe méthyle ou bien $R_1$ et $R_2$ forment ensemble un groupe alkylène en C2-C3.

4. Procédé selon l'une des revendications 1 à 3, caractérisé en ce que l'on utilise un racémate d'un composé de formule II dans laquelle X et Y représentent chacun le chlore ou bien X représente le brome et Y le chlore et $R_1$ et $R_2$ sont tous deux des groupes méthyle.

5. Procédé selon l'une des revendications 1 à 4, caractérisé en ce que l'on fait réagir le racémate d'une cyclobutanone de formule II à une température de 0 à 100°C environ en présence d'un solvant organique inerte et en présence d'eau et d'anhydride sulfureux, l'eau et l'anhydride sulfureux étant utilisés en quantités d'au moins un équivalent molaire par rapport à la base énantiomère.

6. Procédé selon l'une des revendications 1 à 5, caractérisé en ce que la séparation d'un mélange de sels diastéréo-isomères de formule III est réalisée par cristallisation fractionnée en présence d'un solvant organique inerte miscible à l'eau.

7. Procédé selon l'une des revendications 1 à 6, caractérisé en ce que l'on décompose le diastéréo-isomère pur de formule III en une cyclobutanone énantiomère de formule II à une température d'environ 30 à 70°C en présence d'un solvant organique inerte et en présence d'un équivalent molaire d'un acide protonique minéral ou organique.

8. Procédé selon la revendication 1, caractérisé en ce que l'on fait réagir un racémate d'une cyclobutanone de formule II dans laquelle X et Y représentent chacun le chlore ou bien X représente le brome et Y le chlore et $R_1$ et $R_2$ représentent chacun un groupe méthyle, dans un solvant polaire miscible à l'eau pris dans le groupe formé par le méthanol, l'éthanol, l'acétonitrile, le tétrahydrofuranne et le dioxanne à une température de 20 à 60°C en présence d'au moins un équivalent molaire d'eau et d'au moins un équivalent molaire d'anhydride sulfureux avec une base énantiomère prise dans le groupe formé par le R-(−)-2-amino-1-butanol, la (−)-éphédrine, la (+)-éphédrine, la S-(−)-1-phényléthylamine, la R-(+)-1-phényléthylamine et le (+)-thréo-1-(p-nitrophényl)-2-N,N-diméthylaminopropane-1,3-diol, avec formation d'un mélange de sulfonates diastéréo-isomères qu'on sépare par cristallisation fractionnée, on scinde le sel diastéréo-isomère désiré dans l'éthanol ou l'acétonitrile en présence d'acide chlorhydrique avec libération de la cyclobutanone énantiomère qu'on convertit ensuite en présence d'une base à une température de −10 à +10°C en le dérivé d'acide 2-(2',2',2'-trihalogénoéthyl)-cyclopropanecarboxylique correspondant à partir duquel on élimine un halogénure d'hydrogène par chauffage.

9. Composé de formule III

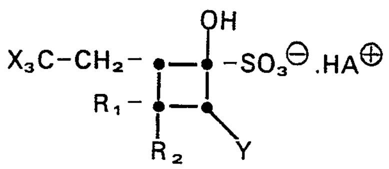

(III)

dans laquelle X représente le chlore ou le brome, l'un des symboles $R_1$ et $R_2$ représente le groupe méthyle et l'autre l'hydrogène ou un groupe méthyle ou bien $R_1$ et $R_2$ forment ensemble un groupe alkylène en C2-C4, A représente une base énantiomère et Y représente le chlore, le brome ou un groupe $-OSO_2R'$, R' représentant un groupe alkyle, halogénoalkyle, benzyle, naphtyle ou phényle éventuellement substitué.

10. Composé de formule III selon la revendication 9, dans lequel A représente le R-(−)-2-amino-1-butanol, la (−)-éphédrine, la (+)-éphédrine, la S-(−)-1-phényléthylamine ou la R-(+)-1-phényléthylamine, X et Y représentent chacun, indépendamment l'un de l'autre, le chlore ou le brome, l'un des symboles $R_1$ et $R_2$ représente le groupe méthyle et l'autre l'hydrogène ou un groupe méthyle ou bien $R_1$ et $R_2$ forment ensemble un groupe alkylène en C2-C3.

11. Composé de formule III selon la revendication 10, dans lequel X et Y représentent chacun le chlore ou bien X représente le brome et Y le chlore et $R_1$ et $R_2$ représentent chacun un groupe méthyle.

12. Procédé de préparation d'un composé de formule III selon la revendication 9, caractérisé en ce que l'on fait réagir un racémate d'une cyclobutanone de formule II

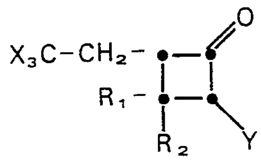

(II)

soit avec le sulfite d'une base énantiomère A, soit en présence d'eau et d'anhydrique sulfureux, avec une base énantiomère A, X, Y, $R_1$, $R_2$ et A ayant les significations indiquées en référence à la formule III.